# EUROPEAN PATENT SPECIFICATION

(11) **EP 4 408 415 B1**
(45) Date of publication and mention of the grant of the patent: **17.06.2026**
(21) Application number: 22800130.1
(22) Date of filing: 30.09.2022
(51) Int. Cl.: A61K 31/352, A61P 27/02, A61K 9/00, C12N 15/00

(54) **ISOFLAVONE DERIVATIVES MODULATING MUTANT BESTROPHIN 1 FOR TREATMENT OF BEST1-RELATED RETINOPATHIES**
ISOFLAVONDERIVATE, DIE MUTANTES BESTROPHIN 1 MODULIEREN, ZUR BEHANDLUNG VON BEST1-VERWANDTEN RETINOPATHIEN
DÉRIVÉS D'ISOFLAVONE MODULANT LA BESTROPHINE 1 MUTANTE POUR LE TRAITEMENT DES RÉTINOPATHIES LIÉES À BEST1

(30) Priority: 30.09.2021 EP 21200142
(43) Date of publication of application: 07.08.2024
(73) Proprietor: Universität Regensburg, 93053 Regensburg (DE)
(72) Inventor: MILENKOVIC, Andrea, 93096 Köfering (DE); AMSLINGER, Sabine, 85609 Aschheim (DE); WEBER, Bernhard, 93083 Obertraubling (DE)
(74) Representative: Drexl, Janna
(86) International application number: PCT/EP2022/077267
(87) International publication number: WO 2023/052579

(56) References cited:
- WO-A1-2020/140007
- WO-A1-2021/198254
- WO-A1-99/18953
- WO-A2-2007/035121
- MARYAM ESKANDARI MEHRABADI ET AL: "Effect of Biochanin A on Retina Levels of Vascular Endothelial Growth Factor, Tumor Necrosis Factor-Alpha and Interleukin-1Beta in Rats With Streptozotocin-Induced Diabetes", CANADIAN JOURNAL OF DIABETES, vol. 42, no. 6, 3 April 2018 (2018-04-03), pages 639 - 644, XP055729954, ISSN: 1499-2671, DOI: 10.1016/j.jcjd.2018.03.008
- ROSEMARY BURGESS ET AL: "Biallelic Mutation of BEST1 Causes a Distinct Retinopathy in Humans", THE AMERICAN JOURNAL OF HUMAN GENETICS, vol. 82, no. 1, 1 January 2008 (2008-01-01), US, pages 19 - 31, XP055680820, ISSN: 0002-9297, DOI: 10.1016/j.ajhg.2007.08.004

## Description

The present invention refers to a compound as defined in formula (I) or a salt thereof for use in a method of treating or preventing Bestrophin 1 (BEST1)-related retinopathies such as autosomal dominant Best vitelliform macular dystrophy. The present invention also refers to a pharmaceutical composition comprising a compound as defined in formula (I) or a salt thereof and a pharmaceutical acceptable excipient and/or carrier for use in a method of treating or preventing BEST1-related retinopathies such as autosomal dominant Best vitelliform macular dystrophy (BVMD). In addition, the present invention refers to a pharmaceutical pack comprising one or more compartments, wherein at least one compartment comprises a compound as defined in formula (I) or a salt thereof or the pharmaceutical composition of the present invention for use according to the present invention. Also, the present invention refers to eye drops, an eye ointment, a skin ointment, a skin gel, a transdermal patch or an implantable device, in particular a micro-drug delivery system, comprising (i) a compound as defined in formula (I) or (ii) a pharmaceutical composition according to the present invention for use according to the present invention.

*BEST1* (MIM 607854) belongs to the bestrophin family of four evolutionarily related genes (BEST1 - 4), that each encode an integral membrane protein. In humans, they function as calcium-activated anion channels although each is specific in terms of gene regulation and tissue distribution. Mutations in *BEST1* which localizes most prominently to the basolateral plasma membrane of the human retinal pigment epithelium (RPE) in the back of the eye are linked to at least four distinct retinopathies, the so called bestrophinopathies, including the autosomal dominant BVMD (MIM 153700) (Marquardt et al., 1998; Petrukhin et al., 1998), the autosomal dominant vitreoretinochoroidopathy (ADVIRC; MIM 193220) (Yardley et al., 2004) as well as the autosomal recessive bestrophinopathy (ARB; MIM 611809) (Burgess et al., 2008). Finally, a phenotype known as pattern dystrophy can be separated from the typical BVMD and is inherited as an autosomal dominant trait with a specific single mutation in BEST1, p.(Ala243Val) (Boon et al. 2009; Khan et al. 2018).

BVMD is the most common pathology of the bestrophinopathies with an estimated prevalence between 1:5 000 and approximately 1:50 000. It is a progressive disorder with typical early onset. The disease is characterized by an accumulation of lipofuscin-like material that resembles an "egg yolk (= vitelliform) in the macular area of the posterior pole. Later, the disintegration of lesions leads to atrophy of the RPE/photoreceptor complex and consequently to vision impairment. An important diagnostic feature is an abnormal Arden ratio (light peak/dark trough ratio) in the electro-oculogram (EOG) response. In contrast to healthy persons Best macular dystrophy patients reveal a reduction in the slow light peak rise, a component thought to reflect an increase in chloride conductance across the basolateral membrane of the RPE.

To date, more than 250 independent disease-causing mutations in the *BEST1* gene have been reported in the Human Gene Mutation Database. These mutations affect BEST1 localization, protein stability and ion gating properties. As a consequence, these functional impairments result in loss of BEST1 chloride transport function.

X-ray structures of chicken BEST1-Fab complexes revealed that the eukaryotic BEST1 channel is a pentameric structure composed of five homomeric BEST1 subunits forming a long 95 Å pore just wide enough for a dehydrated chloride ion to pass. Two constrictions in the pore, the so-called "neck" and the "aperture", are responsible for ion gating and ion selectivity, respectively. Cryo-electron microscopy and electrophysiological recordings revealed that the introduction of various mutations into the chicken BEST1 structure causes (i) changes in the relative permeabilities among anions, (ii) produces channels with dramatically altered gating properties and (iii) diminishes channel inactivation.

So far, the inventors of the present invention have provided Biochanin A derivatives for the treatment for BVMD and other BEST1-linked retinopathies in PCT/EP2021/058287 filed on 30 March 2021 and published in WO 2021/198254. Said Biochanin A derivatives can be used to specifically address amelioration of impaired BEST1 channel functionality. They are able to restore (BEST1-mediated) anion transport to address the primary defect in BEST1-associated bestrophinopathies such as BEST1-related BVMD, namely impairment of chloride conductance. However, there is still a need of providing further compounds which are able to restore (BEST1-mediated) anion transport to address the primary defect in BEST1-associated bestrophinopathies such as BESTl-related BVMD, in particular autosomal dominant BVMD, namely impairment of chloride conductance. In particular, clinical evaluation of the compounds provided must demonstrate which compounds are most effective without causing undesirable side effects.

WO 2020/140007 A1 discloses a method of treating a retinal degenerative disorder associated with a BEST1 dominant mutation in a subject, such as a bestrophinopathy selected from the group consisting of: Best vitelliform macular dystrophy (BVMD), adult-onset vitelliform dystrophy (AVMD), autosomal dominant vitreoretinochoroidopathy (ADVIRC), and retinitis pigmentosa (RP) by administering a nucleic acid molecule encoding wild-type BEST1. WO 2020/140007 A1 does not disclose the administration of a small molecule as defined in formula (I) of the present invention.

Maryam Eskandari Mehrabadi et al. (Canadian Journal of Diabetes, vol. 42, no. 6, 3 April 2018, pages 639-644) discloses biochanin A as improving diabetic retinopathy but not BEST1-related retinopathies.

WO 99/18953 A1 discloses flavones and/or isoflavones to activate chloride transport in epithelial tissues of the airways, the intestine, the pancreas and other exocrine glands and for cystic fibrosis therapy. Exemplary compounds are biochanin A, genistein and many others. However, WO 99/18953 A1 does neither disclose the specific derivatives as defined in formula (I) of the present invention nor does it address BEST1 or the treatment of retinopathies.

WO 2007/035121 A2 discloses isoflavone derivatives encompassing derivatives according to formula (I) of the present invention for use in the treatment of diseases, at the base of which lies an excessive production or storage of glycosaminoglycans, especially for treatment of mucopolysaccharidoses. WO 2007/035121 A2 does not address BEST1-related retinopathies.

Rosemary Burgess et al. (The American Journal of Human Genetics, vol. 82, no. 1, 1 January 2008, pages 19-31) describes the relation between BEST1 mutations and related retinopathies but does not address the treatment of BEST1 related retinopathies, and does not disclose compounds as defined in formula (I) of the present invention.

The problem to be solved by the present invention was thus to provide compounds which are able to restore (BEST1-mediated) anion transport to address the primary defect in BEST1-associated bestrophinopathies such as BEST1-related BVMD, in particular autosomal dominant BVMD, namely impairment of chloride conductance.

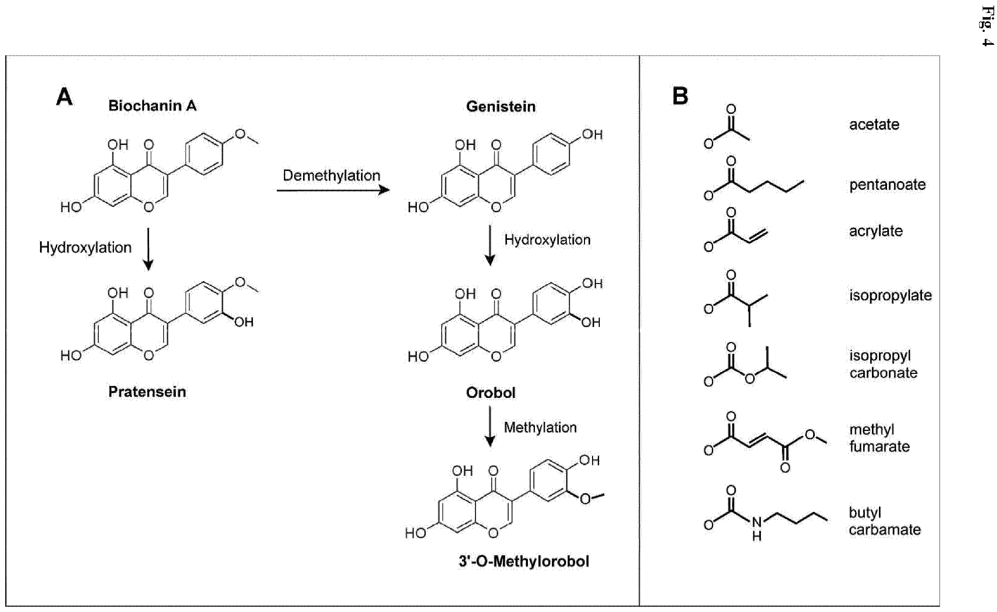

Isoflavones are a major group of phytoestrogens that have been the focus of a number of studies in recent years. Biochanin A, a natural isoflavone found in red clover flowers, is the methylated structural analog of Genistein (Fig. 4A), which, unlike Biochanin A, is present in large amounts in soy plants and soy-based foods. Both isoflavones are major ingredients in a variety of dietary supplements used to alleviate postmenopausal symptoms. In addition to their estrogenic activity, epidemiological and clinical studies suggest health benefits of Genistein in many chronic diseases, such as cardiovascular disease and diabetes. Pratensein is a hydroxylated structural analog of Biochanin A, also present in red clover, (Fig. 4A) and may have effects on the prevention of atherosclerosis. 3'-O-Methylorobol is a methylated structural derivative of Orobol (Fig. 4A) that exerts potential analgesic properties and exhibites moderate antioxidant activity.

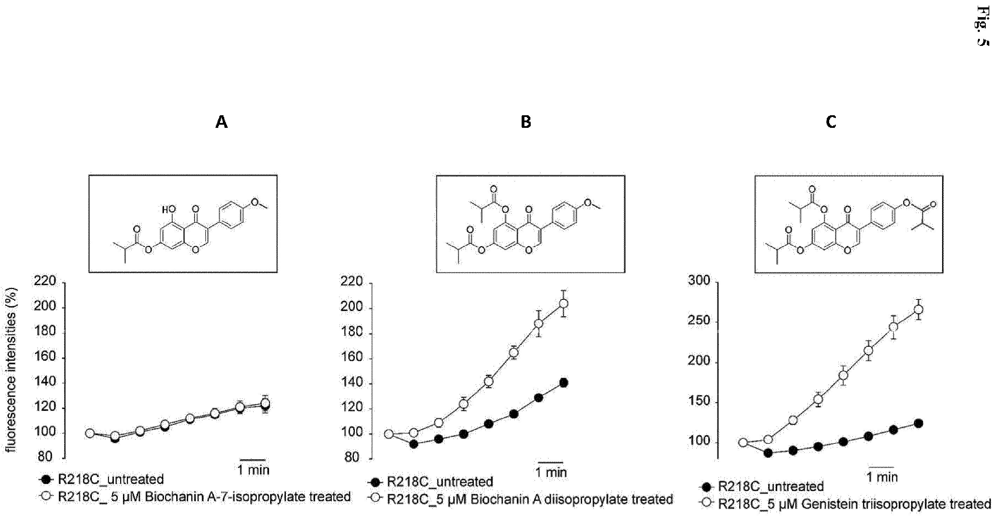

From the structural formula of Genistein, Orobol, Pratensein and 3'-O-Methylorobol, it is evident that the phenolic groups at carbon atoms C-5, C-7, and C-3' or C-4' provide an opportunity for derivatization to enhance the biological activity of the isoflavone backbones, by increasing the cellular uptake rate and its biological availabity and by prolonging its stability. Esters of bioactive phenolics are known to increase the permeability by increasing lipophilicity, thereby enhancing oral bioavailability and metabolic stability. To expand the repertoire of isoflavone compounds with improved pharmacological properties and increased substance activity, new substance derivatives were developed in a systematic ester-prodrug approach with 7 different functional groups (Fig. 4B) and the isoflavone backbones Genistein, Pratensein, 3'-O-Methylorobol and Orobol. After synthesis, the new ester-isoflavone derivatives were tested for their efficacy in the MDCKII and hiPSC-RPE cell model, respectively, using the YFP halide transport assay. Our findings indicate amongth others that bilateral esterification at the C-5 and C-7 carbon atom was associated with a very strong improvement in channel function of mutant BEST1 (Fig. 5B), whereas a single esterification at the C-7 position showed only minimal or no effect (Fig. 5A). The activity could even be increased by an additional esterification at the C-4' and C-3' position, respectively (Fig. 5C).

The problem underlying the present invention is solved by the subject matter defined in the claims.

The following figures serve to illustrate the invention.

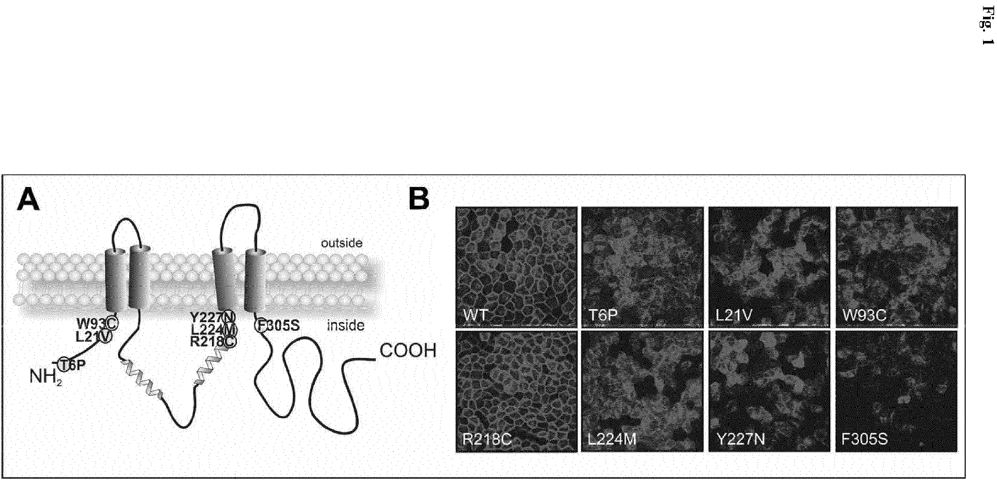

Figure 1 depicts the generation of MDCKII stable cell lines expressing normal and mutant BEST1. (A) Seven disease associated mutations representing the hotspot mutational regions of the protein were selected as shown in Fig. 1A. Normal BEST1 sequences were cloned into pcDNA3 and mutants were generated by site-directed mutagenesis. (B) Normal (WT) and mutant (p.(T6P), p.(L21V), p.(W93C), p.(R218C), p.(L224M), p.(Y227N), p.(F305S)) BEST1 constructs were stably transfected in MDCKII cells. Single clones were isolated in a selection medium containing G418. Stable cell lines were grown 7 days on coverslips to achieve cell polarity and BEST1 localization was determined by immunocytochemistry.

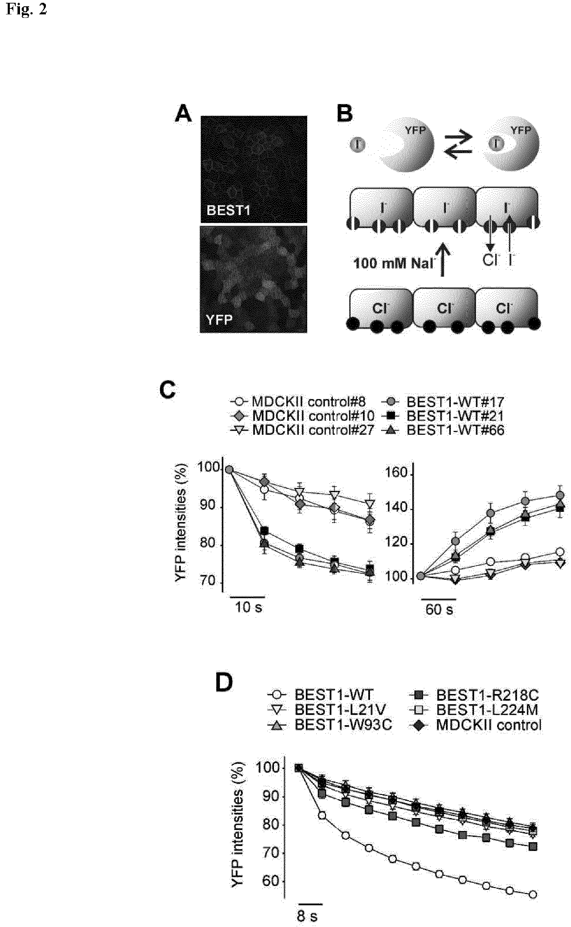

Figure 2 shows the analysis of BEST1-mediated halide transport in MDCKII cell lines stably co-expressing YFP and normal or mutant BEST1. (A) Representative fluorescence images of MDCKII cell line stably co-expressing wildtype BEST1 and YFP. (B) Graphical illustration of the principle behind the halide transport assay. When iodide (I⁻) enters the cell through open anion channels, the YFP fluorescence stably expressed in the MDCKII cells is reduced. (C) Time course of cell fluorescence signals in MDCKII cells expressing wildtype BEST1 and untransfected controls (as indicated). Cells were incubated in 40 mM Cl⁻ (= 100%) and then Cl⁻ was replaced by I⁻ (n=6) (left graph). Cells were incubated in 40 mM I⁻ (=100%) and then I⁻ was replaced by Cl⁻ (right graph). (D) Same experiments as in (C). MDCKII cells expressing no, wildtype or mutant BEST1 (as indicated) (n=6).

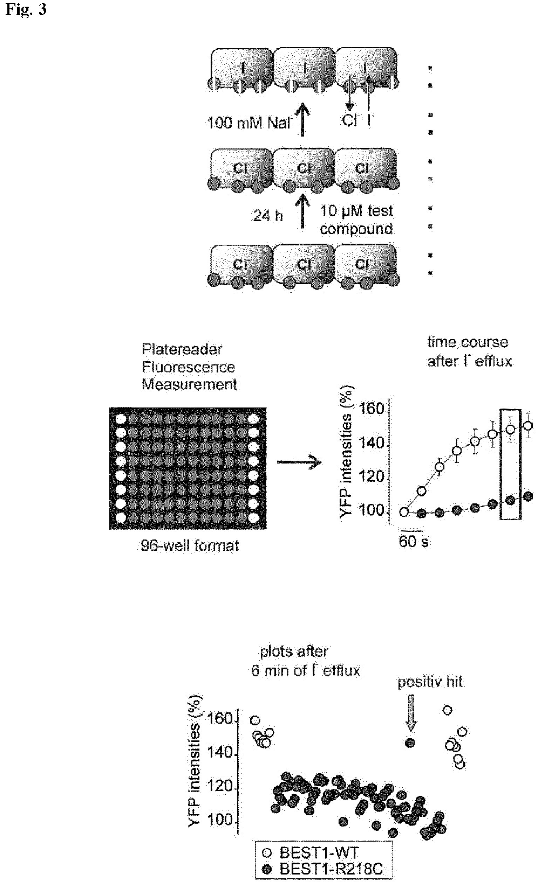

Figure 3 displays the schematic of the drug screening approach. Stably transfected MDCKII cells co-expressing YFP and BEST1 wildtype (BEST1-WT; white circles) or mutant p.(R218C) (BEST1-R218C; grey circles) were cultivated in black 96-well plates for six days. After addition of a test compound, cells were incubated in 40 mM I⁻ for 6 min and then replaced by Cl⁻. Each of the grey wells in the eight rows was loaded with a different compound and values of YFP intensities after Cl⁻ application (boxed) were plotted.
Figure 4 depicts the development of new isoflavone derivatives. Shown is the molecular structure of main metabolites and structural analogs of Biochanin A and Genistein, respectively, (Fig. 4A) and selected functional groups for ester formation at the hydroxy group position of the isoflavones indicated (Fig. 4B).
Figure 5 shows the effect of esterification on BEST1 anion transport function in MDCKII cells. While replacement of a single hydroxy group at position C-7 of Biochanin A by the functional group isopropyl (Biochanin A-7-isopropylate) showed no effect in comparison to untreated cells (Fig. 5A), anion transport activity was stronly increased for mutant BEST1-R218C upon addition of Biochanin A diisopropylate, where two hydroxy groups at position C-7 and C-5 were replaced (Fig. 5B). The introduction of 3 isopropyl esters into the isoflavone backbone further increased this activity (Fig. 5C).

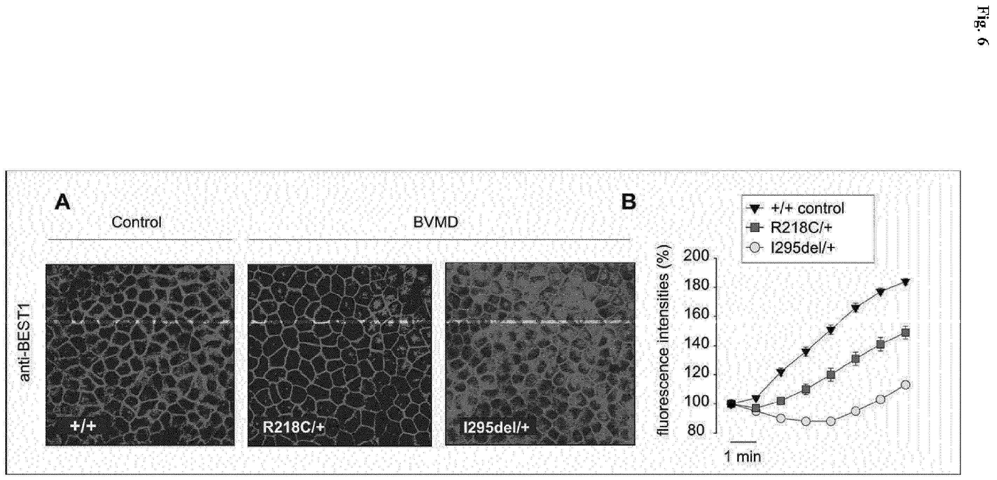

Figure 6 shows the analysis of BEST1-mediated halide transport in iPSC-RPEs from of a healthy donor (hiPSC-RPE +/+) and two Best macular dystrophy patients heterozygous for BEST1 mutation p.(R218C) (hiPSC-RPE-R218C/+) and p.(I295del) (hiPSC-RPE-I295del/+), respectively. (A) Representative fluorescence images of hiPSC-RPE cell lines +/+, R218C/+ and I295del/+ using a specific BEST1 antibody. While hiPSC-RPEs from mutant R218C/+ and control show correct localization of BEST1 on the plasma membrane, a large proportion of BEST1 in hiPSC-RPE-1295del/+ reveals mislocalization in the cytoplasm. (B) Time course of cell fluorescence signals in hiPSC-RPE cell lines +/+, R218C/+ and I295del/+ as indicated. Cells were incubated in 40 mM I⁻ (= 100%) and then I⁻ was replaced by Cl⁻ (n=6).

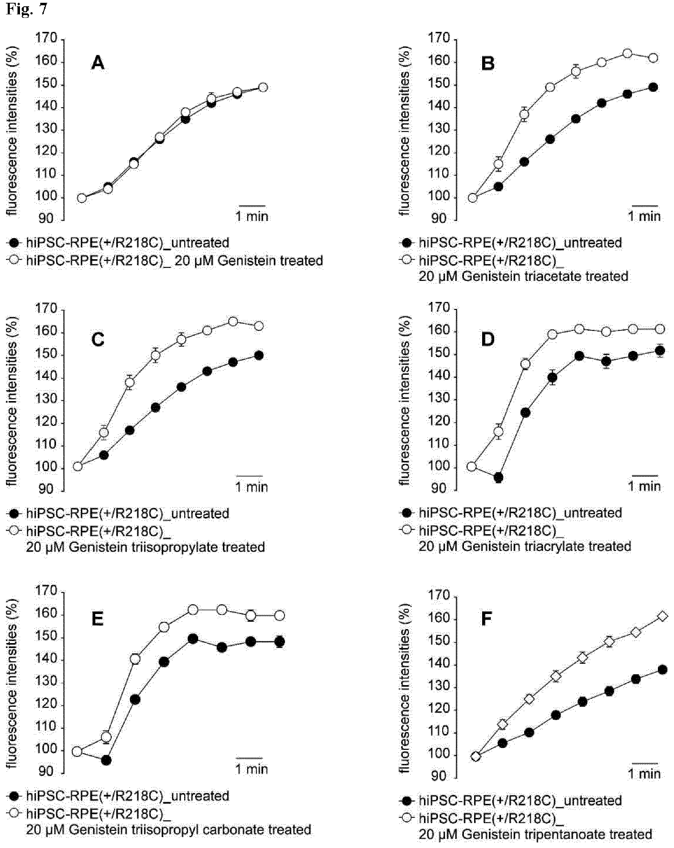

Figure 7 demonstrates the effect of Genistein (Fig. 7A) and several Genistein derivatives, namely Genistein triacetate (Fig. 7B), Genistein triisopropylate (Fig. 7C), Genistein triacrylate (Fig: 7D), Genistein triisopropyl carbonate (Fig. 7E) and Genistein tripentanoate (Fig. 7F) on BEST1 anion transport function in patient-derived hiPSC-RPE cells in comparison to untreated hiPSC-RPE cells. While Genistein (Fig. 7A) showed no effect upon addition of compound delivery in comparison to untreated cells, anion transport activity was strongly increased in R218C/+ hiPSC-RPE cells upon addition of Genistein triacetate (Fig. 7B), Genistein triisopropylate (Fig. 7C), Genistein triacrylate (Fig. 7D), Genistein triisopropyl carbonate (Fig. 7E) and Genistein tripentanoate (Fig. 7F).

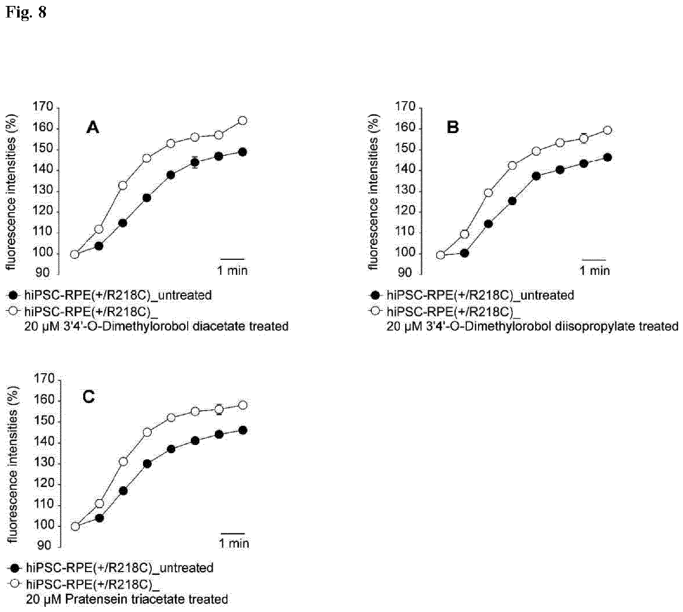

Figure 8 reveals the effect of two 3',4'-*O*-Dimethylorobol derivates and a Pratensein derivative, namely 3',4'-*O*-Dimethylorobol diacetate (Fig. 8A), 3',4'-*O*-Dimethylorobol diisopropylate (Fig. 8B) and Pratensein triacetate (Fig. 8C) on BEST1 anion transport function in patient-derived hiPSC-RPE cells in comparison to untreated hiPSC-RPE cells. All of 3',4'-*O-*Dimethylorobol diacetate (Fig. 8A), 3',4'-*O* -Dimethylorobol diisopropylate (Fig. 8B) and Pratensein triacetate (Fig. 8C) increased anion transport activity in R218C/+ hiPSC-RPE cells upon addition of respective compound delivery in comparison to untreated cells significantly.

The term "Genistein" as used herein refers in particular to a compound of formula (II):

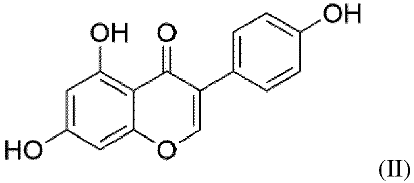

The term "Genistein" as used herein can synonymously be used with the terms "C₁₅H₁₀O₅" or "4',5,7-Trihydroxyisoflavone" or "5,7-dihydroxy-3-(4-hydroxyphenyl)-4*H*-chromen-4-one".

The term "Pratensein" as used herein refers in particular to a compound of formula (III):

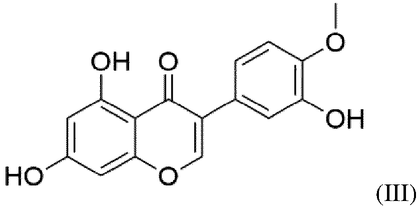

The terrm "Pratensein" as used herein can synonymously be used with the terms "5,7-Dihydroxy-3-(3-hydroxy-4-methoxyphenyl)-4*H*-1-benzopyran-4-one" or "4'-Methoxy-3',5,7-trihydroxyisoflavone" or "3'-Hydroxybiochanin A" or "C₁₆H₁₂O₆".

The term "Orobol" as used herein refers in particular to a compound of formula (IV):

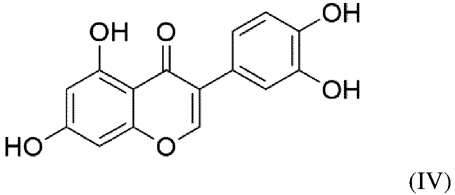

The term "Orobol" as used herein can synonymously be used with the terms "3',4',5,7-Tetrahydroxyisoflavone" or "3-(3,4-Dihydroxyphenyl)-5,7-dihydroxy-4*H*-1-benzopyran-4-one" or "C₁₅H₁₀O₆".

The term "3',4'-*O*-Dimethylorobol" as used herein refers in particular to a compound of formula (V):

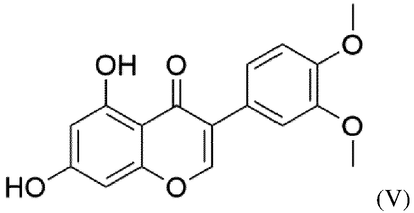

The term "3',4'-O-Dimethylorobol" as used herein can synonymously be used with the terms "5,7-Dihydroxy-3',4'-dimethoxyisoflavone" or "C₁₆H₁₂O₆".

The term "3'-O-Methylorobol" as used herein refers in particular to a compound of formula (VI):

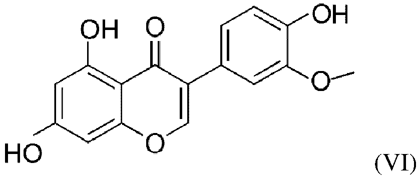

The term "3'-O-Methylorobol" as used herein can synonymously be used with the terms "C₆H₁₂O₆" or "5,7-dihydroxy-3-(4-hydroxy-3-methoxyphenyl)chromen-4-one" or "4',5,7-trihydroxy-3'-methoxyisoflavone".

The term "derivative" as used herein in the context of any of the chemical compounds described herein relates to a chemical molecule having a structure related to said chemical compound of the present invention. Preferably, a derivative still has the basic structure of the respective chemical compound and varies only at the variable residues. More preferably, a derivative still has the basic structure of the respective chemical compound as outlined each in formula (I) to (XI) and varies only at positions R1, R2, R3, R4 and/or X.

The term "BEST1-related retinopathies" relates to retinopathies caused by mutations in the *BEST1* gene (MIM 607854). BEST1 localizes most prominently to the basolateral plasma membrane of the RPE in the back of the eye. Mutations in *BEST1* affect BEST1 localization, protein stability and ion gating properties. As a consequence, these functional impairments result in loss of BEST1 channel function, in particular anion transport function, more preferably chloride transport function, which causes a damage of the retina of the eye.

The term "BEST1" as used herein is a shortcut for bestrophin-1. It can synonymously be used with the term "VMD2". BEST1 belongs to the bestrophin family of four evolutionary related genes (BEST1 - 4), that encode for integral membrane proteins. In humans, they function in particular as calcium-activated anion channels although each is specific in terms of gene regulation and tissue distribution of the protein. Wildtype BEST1 (Homo sapiens bestrophin 1, BEST1) has preferably an amino acid sequence as shown in SEQ ID NO: 1. Wildtype BEST1 (Homo sapiens bestrophin 1, BEST1) is preferably encoded by a nucleic acid sequence as shown in SEQ ID NO: 2.

The term "bestrophinopathy" refers to a group of phenotypes of degenerative retinal diseases caused by one or more mutations in the *BEST1* gene, in particular one mutation (in the case of autosomal dominant inheritance) or two mutations (in case of autosomal recessive inheritance) in the BEST1 gene.

The term "Best vitelliform macular dystrophy (BVMD)" as used herein may also be called "Best macular dystrophy", "vitelliform macular dystrophy-2 (VMD2)", or short "Best disease". It is a hereditary retinal dystrophy involving the RPE/photoreceptor complex, characterized in early stages of the diseases by the appearance of yellow "egg-yolk"-like lesions in the macular area. It is the most common phenotype of the bestrophinopathies. Subjects suffering from autosomal dominant Best vitelliform macular dystrophy have a mutation in the *BEST1* gene which leads to a loss of channel function and eventually retinal degeneration.

The term "autosomal dominant vitreoretinochoroidopathy (ADVIRC)" refers to a chorioretinal pigmentary disorder affecting the peripheral retina. According to the state of the art in particular the five *BEST1* gene mutations p.(Gly83Asp), p.(Val86Met), p.(Val235Ala), p.(Tyr236Cys), and p.(Va1239Met) are causative of ADVIRC. The disease is classically characterized by a peripheral retinal circumferential hyperpigmented band with a well-defined posterior demarcation and can be associated with developmental ocular anomalies such as microcornea, microphthalmos, angle closure glaucoma, and cataract. The term "autosomal recessive bestrophinopathy (ARB)" refers to a disease which is caused by homozygous or compound heterozygous *BEST1* gene mutations. The main characteristics of ARB are multifocal subretinal deposits outside the macular area, abnormal autofluorescence and subretinal fluid accumulation or macular edema. Heterozygous parents generally show no retinal symptoms.

The term "pattern dystrophy" as used herein refers to a special form of autosomal dominant Best macular dystrophy, which is in particular associated with a p.(Ala243Val) mutation. In this form the vitelliform lesions are missing and, instead, patterned pigment changes are formed. The course of disease is usually mild. Preferably, the term "pattern dystrophy" as used herein is exclusively associated with a p.(Ala243Val) mutation.

Any mutation as described in the present disclosure refers to wildtype BEST1 as shown in SEQ ID NO: 1.

The term "improving chloride conductance" refers to an increased level of measurable chloride conductance in a given assay in the presence of a candidate compound relative to the measurable level of chloride conductivity in the absence of the candidate compound, when tested under the same conditions. The chloride conductance is improved according to the invention if it is enhanced at least about 5%, 10%, 20%, 30%, 40%, 50%, 60%, 70%, 80%, 90%, 95%, 100%, 150%, 200%, 250%, 300% or more than in the absence of the candidate compound. An improved chloride conductance represents an improved anion transport activity. Improvement of anion transport activity can e.g. be tested as described in Example 7 or 8. Thus, an improved chloride conductance can be evaluated e.g. by performing a test as demonstrated in Example 7 or 8.

The term "restoring BEST1 channel function" particularly refers to a repair of the BEST1 anion transport function, in particular the chloride transport function, if the BEST1 channel function is impaired e.g. by one or more mutations in the BEST1 gene. The repair of BEST1 channel function can be determined in a given assay in the presence of a candidate compound relative to the measurable level of BEST1 channel function in the absence of the candidate compound, when tested under the same conditions and compared to BEST1 channel function of a control having no mutations in the BEST1 gene. The BEST1 channel function is restored according to the invention if at least about 5%, 10%, 20%, 30%, 40%, 50%, 60%, 70%, 80%, 90%, 95% or more of BEST1 channel function can be restored in the presence of the candidate compound. The term "restoring BEST1 channel function" may also be used if the BEST1 channel function is improved at least about 100%, 150%, 200%, 250%, 300% or more in the presence of the candidate compound.

The term "comprising" as used herein shall not be construed as being limited to the meaning "consisting of" (i. e. excluding the presence of additional other matter). Rather, "comprising" implies that optionally additional matter may be present. The term "comprising" encompasses as particularly envisioned embodiments falling within its scope "consisting of" (i. e. excluding the presence of additional other matter) and "comprising but not consisting of" (i. e. requiring the presence of additional other matter), with the former being more preferred.

In a first object of the present invention it is envisaged to provide a modulator of the BEST1 chloride channel, which is able to restore BEST1 channel function. Said modulator is preferably an isoflavone derivative that modulates mutant bestrophin 1. In particular, the present invention provides a compound having the formula (I) or a salt thereof: 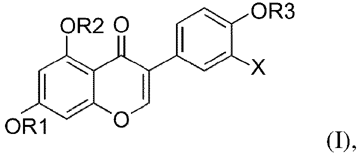 wherein
OR1 is an acetate, a pentanoate, an isopropylate, an acrylate, a methyl fumarate, a butyl carbamate or an isopropyl carbonate
OR2 is an acetate, a pentanoate, an isopropylate, an acrylate, a methyl fumarate, a butyl carbamate or an isopropyl carbonate,
OR3 is an acetate, a pentanoate, an isopropylate, an acrylate, a methyl fumarate, a butyl carbamate, or an isopropyl carbonate, or R3 is a methyl, and
X is a hydrogen or OR4, wherein OR4 is an acetate, a pentanoate, an isopropylate, an acrylate, a methyl fumarate, a butyl carbamate, or an isopropyl carbonate, or R4 is a methyl,
for use in a method of treating or preventing BEST1-related retinopathies such as BVMD, in particular autosomal dominant BVMD.

In a preferred embodiment of the compound having formula (I) as defined above, R3 is not methyl if X is a hydrogen. Thus, in a preferred embodiment the compound having formula (I) is as defined above under the provision that when X is a hydrogen, R3 is not a methyl. This means that in a preferred embodiment, OR3 is an acetate, a pentanoate, an isopropylate, an acrylate, a methyl fumarate, a butyl carbamate or an isopropyl carbonate if X is hydrogen.

Preventing BEST1-related retinopathies such as BVMD comprises preferably delaying the onset and/or progression of BEST1-related retinopathies such as BVMD. Treating BEST1-related retinopathies such as BVMD may also comprise delaying the progression of BEST1-related retinopathies such as BVMD or preventing the reoccurrence of symptoms of the disease after the disease has previously been treated. The compounds according to the present invention are able to modulate the BEST1 transport channel, in particular by improving the chloride conductance across the basolateral membrane of the RPE of the subject thereby restoring BEST1 channel function. In particular, the compounds for use according to the present invention are able to compensate reduced anion transport activity of BEST1 by enhancing residual BEST1 channel function or activating other - yet undefined - anion channels in the RPE of subjects.

The compounds solving the problem underlying the present invention is a compound according to formula (I): 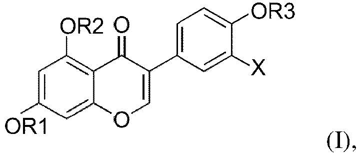
wherein X is a hydrogen or OR4, and
each of R1, R2, R3 and R4 may independently be selected from the group consisting of any one of the following residues:

| **Ri, i=1, 2, 3 or 4** | **ORi group** | **Ri, i=1, 2, 3 or 4** | **ORi group** |
|---|---|---|---|
| 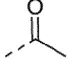 | acetate | 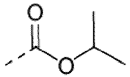 | isopropyl carbonate |
| 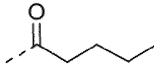 | pentanoate | | |
| | | 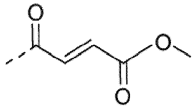 | methyl fumarate |
| 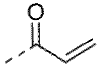 | acrylate | | |
| 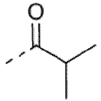 | isopropylate | 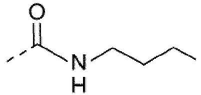 | butyl carbamate |

and R3 and R4 may also independently be a methyl group, preferably under the provision that X is not a hydrogen if R3 is a methyl.

In a particularly preferred embodiment of the present invention, R1 and R2 in formula (I) are the same.

In a further preferred embodiment of the present invention, X is a hydrogen. If X is a hydrogen, the compound as shown in formula (I) is a genistein derivative having the following formula (VII): 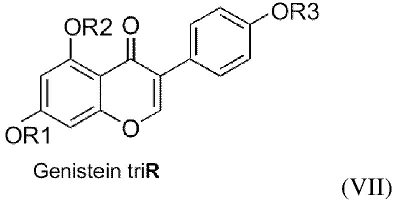 R1, R2 and R3 are as defined above. Preferably R1 and R2 are the same. In a further preferred embodiment R1, R2 and R3 are the same. R3 is preferably not methyl.

Genistein triacetate, Genistein tripentanoate, Genistein triisopropylate, Genistein triacrylate, Genistein tri(methyl fumarate), Genistein tri(butyl carbamate) and Genistein tri(isopropyl carbonate), represent particularly preferred genistein derivatives for use according to the present invention.

In a further preferred embodiment of the present invention, X is OR4 and R4 is a methyl. If X is OR4 and R4 is a methyl, the compound as shown in formula (I) is a 3'-O-Methylorobol derivative having the following formula (VIII): 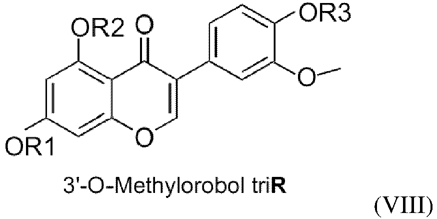 R1, R2 and R3 are as defined above. Preferably R1 and R2 are the same. In a further preferred embodiment R1, R2 and R3 are the same.

3'-O-Methylorobol triacetate, 3'-O-Methylorobol tripentanoate, 3'-O-Methylorobol triisopropylate, 3'-O-Methylorobol triacrylate, 3'-O-Methylorobol tri(methyl fumarate), 3'-O-Methylorobol tri(butyl carbamate) and 3'-O-Methylorobol tri(isopropyl carbonate) represent particularly preferred 3'-O-Methylorobol derivatives for use according to the present invention.

In a further preferred embodiment of the present invention, R3 is a methyl, X is OR4 and R4 is a methyl. If R3 is a methyl, X is OR4 and R4 is a methyl, the compound as shown in formula (I) is a 3',4'-O-Dimethylorobol derivative having the following formula (IX): 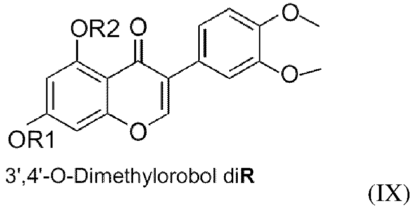 R1 and R2 are as defined above and are preferably the same.

3',4'-O-Dimethylorobol diacetate, 3',4'-O-Dimethylorobol dipentanoate, 3',4'-O-Dimethylorobol diisopropylate, 3',4'-O-Dimethylorobol diacrylate, 3',4'-O-Dimethylorobol di(methyl fumarate), 3',4'-O-Dimethylorobol di(butyl carbamate) and 3',4'-O-Dimethylorobol di(isopropyl carbonate) represent particularly preferred 3',4'-O-Dimethylorobol derivatives for use according to the present invention.

In a further preferred embodiment of the present invention, X is OR4. If X is OR4, the compound as shown in formula (I) is an Orobol derivative having the following formula (X): 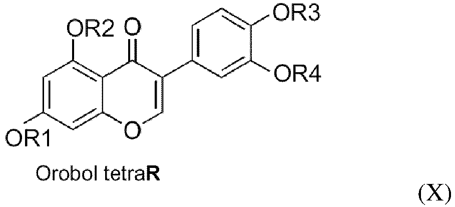 R1, R2, R3 and R4 are as defined above. Preferably, R1 and R2 are the same. In a further preferred embodiment R3 and R4 are the same. In a further preferred embodiment R1, R2 and R3 are the same. In a further preferred embodiment R1, R2 and R4 are the same. In a further preferred embodiment R1, R2, R3 and R4 are the same.

3',4',5,7-O-Orobol tetraacetate, 3',4',5,7-O-Orobol tetrapentanoate, 3',4',5,7-O-Orobol tetraisopropylate, 3',4'5,7-O-Orobol tetraacrylate, 3',4',5,7-O-Orobol tetra(methyl fumarate), 3',4',5,7-O-Orobol tetra(butyl carbamate) and 3',4',5,7-O-Orobol tetra(isopropyl carbonate) represent particularly preferred Orobol derivatives for use according to the present invention.

In a further preferred embodiment of the present invention, R3 is a methyl and X is OR4. If R3 is a methyl and X is OR4, the compound as shown in formula (I) is a Pratensein derivative having the following formula (XI): 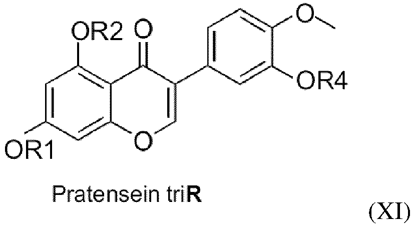 R1, R2 and R4 are as defined above. Preferably R1 and R2 are the same. In a further preferred embodiment R1, R2 and R4 are the same.

Pratensein triacetate, Pratensein tripentanoate, Pratensein triisopropylate, Pratensein triacrylate, Pratensein tri(methyl fumarate), Pratensein tri(butyl carbamate) and Pratensein tri(isopropyl carbonate) represent particularly preferred Pratensein derivatives for use according to the present invention.

In a preferred embodiment of the present invention, the compound or a salt thereof for use according to the present invention is a compound selected from the group consisting of: Genistein triacetate, Genistein tripentanoate, Genistein triisopropylate, Genistein triacrylate, Genistein tri(methyl fumarate), Genistein tri(butyl carbamate), Genistein tri(isopropyl carbonate), Pratensein triacetate, Pratensein tripentanoate, Pratensein triisopropylate, Pratensein triacrylate, Pratensein tri(methyl fumarate), Pratensein tri(butyl carbamate), Pratensein tri(isopropyl carbonate), 3'-*O*-Methylorobol triacetate, 3'-*O*-Methylorobol tripentanoate, 3'-*O*-Methylorobol triisopropylate, 3'-*O*-Methylorobol triacrylate, 3'-*O-*Methylorobol tri(methyl fumarate), 3'-*O*-Methylorobol tri(butyl carbamate), 3'-*O-*Methylorobol tri(isopropyl carbonate), 3',4'-*O*-Dimethylorobol diacetate, 3',4'-*O-*Dimethylorobol dipentanoate, 3',4'-*O-*Dimethylorobol diisopropylate, 3',4'-*O-*Dimethylorobol diacrylate, 3',4'-*O-*Dimethylorobol di(methyl fumarate), 3',4'-*O-*Dimethylorobol di(butyl carbamate), 3',4'-*O*-Dimethylorobol di(isopropyl carbonate), 3',4',5,7-*O*-Orobol tetraacetate, 3',4',5,7-*O*-Orobol tetrapentanoate, 3',4',5,7-*O*-Orobol tetraisopropylate, 3',4',5,7-*O*-Orobol tetraacrylate, 3',4',5,7-*O*-Orobol tetra(methyl fumarate), 3',4',5,7-*O*-Orobol tetra(butyl carbamate), 3',4',5,7-*O*-Orobol tetra(isopropyl carbonate).

In a further preferred embodiment of the present invention, the compound or a salt thereof for use according to the present invention is a compound selected from the group consisting of:

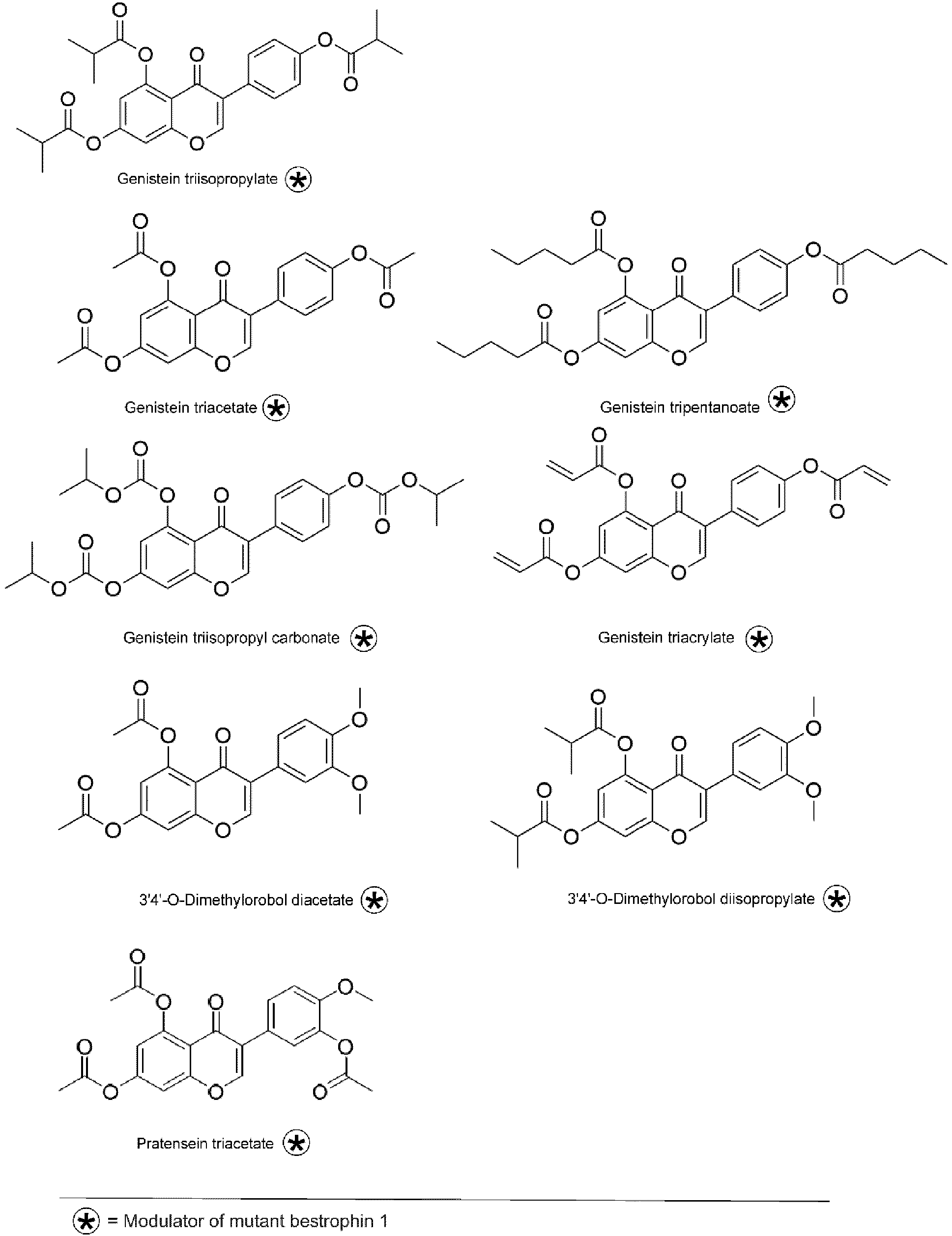

The method of treating, preventing and/or delaying the onset or progression of BEST1-related retinopathies such as BVMD preferably comprises the administration of a compound for use according to the present invention as defined above or a salt thereof to the subject. Preferably, said compound or salt thereof is administered in an amount sufficient to improve chloride conductance across the basolateral membrane of the RPE of the subject. It is preferably administered in an amount sufficient to restore BEST1 channel function. It is preferably administered in an amount sufficient to compensate reduced anion transport activity of BEST1 by enhancing residual BEST1 channel function or activating other - yet undefined - anion channels in the RPE of subjects. In a preferred embodiment the method of treating comprises additionally the step of molecular genetic testing of BEST1. In an especially preferred embodiment, the method comprises the steps of
(i) evaluating the subject's vision before treatment;
(ii) molecular genetic testing of the *BEST1* gene;
(iii) administering a preferred compound as defined above or a salt thereof to the subject; and
(iv) evaluating of subject's vision after step (iii).

Molecular genetic testing of the *BEST1* gene may be performed from DNA extracted from a peripheral blood sample of the subject. Mutation analysis may be done by the Sanger chain termination method, a technique for DNA sequencing based upon the selective incorporation of chain-terminating dideoxynucleotides (ddNTPs) by DNA polymerase during in vitro DNA replication. Specifically, the nucleotide sequence of the 11 coding sequences (exons) and the respective flanking intronic regions (about 20 base pairs each) will be sequenced from the *BEST1* gene of the subject.

The present invention also refers to a pharmaceutical composition comprising a preferred compound as defined above or a salt thereof and a pharmaceutical acceptable excipient and/or carrier for use in a method of treating, preventing and/or delaying the onset or progression of BEST1-related retinopathies as defined above.

The pharmaceutical composition according to the present invention may additionally contain one or more conventional additive(s). Some examples of such additives include a solubilizer such as, for example, glycerol; an antioxidant such as for example, benzalkonium chloride, benzyl alcohol, chloretone or chlorobutanol; and/or an isotonic agent. As a further precaution against oxidation or other spoilage, the pharmaceutical compositions may be stored under a suitable gas, as e.g. nitrogen gas, in vials sealed with impermeable stoppers.

Preferably, the preferred compound as defined above or a salt thereof and/or the pharmaceutical composition of the present invention is for use in a method of treating or preventing BEST1-related retinopathies, in particular the so-called bestrophinopathies. In a preferred embodiment of the present invention said BEST1-related retinopathies or bestrophinopathies result from an alteration of the BEST1 protein which may be caused by a mutation in the BEST1 gene. Preferably, such BEST 1-related retinopathies or bestrophinopathies are caused or characterized by an impaired and/or reduced anion transport activity of BEST1. The BEST1-related retinopathies or bestrophinopathies may be selected from the group consisting of autosomal dominant Best macular dystrophy, vitelliform macular dystrophy-2 (VMD2), autosomal recessive bestrophinopathy, or pattern dystrophy, more preferably from the group consisting of autosomal dominant Best macular dystrophy, vitelliform macular dystrophy-2 (VMD2), autosomal recessive bestrophinopathy, or pattern dystrophy. Treating and preventing of autosomal dominant BVMD is especially preferred. In a preferred embodiment of the present invention, the BEST1-related retinopathies or bestrophinopathies do not comprise autosomal dominant vitreoretinochoroidopathy (ADVIRC). Thus, in a preferred embodiment of the present invention, the preferred compound as defined above or a salt thereof and/or the pharmaceutical composition of the present invention is for use in a method of treating or preventing BEST1-related retinopathies excluding autosomal dominant vitreoretinochoroidopathy (ADVIRC).

The subject to be treated is preferably a human or an animal, in particular a mammal, most preferably a human. The preferred compounds as defined above or a salt thereof and/or the pharmaceutical composition of the present invention may be administered to the subject in need thereof in an effective amount. The effective amount of the compound to be administered can be readily determined by those skilled in the art during pre-clinical trials and clinical trials by methods familiar to physicians and clinicians.

In accordance with all embodiments of the present invention, an effective amount of the preferred compounds as defined above or a salt thereof for use in a method of treating or preventing BEST1-related retinopathies, preferably in a pharmaceutical composition, may be administered to the subject in need thereof by any of a number of well-known methods for administering pharmaceutical compounds. The compound may be administered locally or systemically. The route of administration may be oral, topical, ocular, intraocular, by eye drops, or by intravitreal injection, or any other suitable route of administration.

In another aspect, the present invention relates to a process of preparation of a pharmaceutical composition, said process comprising admixing the compound or a salt thereof and/or the pharmaceutical composition of the present invention with a pharmaceutically acceptable diluent, excipient or carrier.

The present invention also relates to a pharmaceutical pack comprising one or more compartments, wherein at least one compartment comprises a preferred compound as defined above or salt thereof or the pharmaceutical composition of the present invention.

In a preferred embodiment the compound or a salt thereof or the pharmaceutical composition according to the present invention is formulated for topical administration, in particular for topical administration to the eye, in particular for intraocular administration. Preferably it is formulated in form of eye drops, an eye ointment or an implantable device. Alternatively, the compound or a salt thereof or the pharmaceutical composition according to the present invention may be formulated for topical administration to the skin e.g. in form of an ointment, a gel, a film, a transdermal patch or similar drug-containing composite devices, in particular a microporous polymer film membrane or hydroalcoholic gel base, for efficient and controlled transdermal drug delivery. Alternatively, the compound or a salt thereof or the pharmaceutical composition according to the present invention may be formulated for oral administration e.g. in form of a tablet, capsule, dragée or pill but also in form of an injectable solution or any other medical reasonable galenic formulation. Preferably, the galenic formulation may comprise suitable carriers, stabilizers, flavourings, buffers or other suitable reagents.

Thus, the present invention also refers to eye drops or an eye ointment comprising a preferred compound as defined above or a salt thereof and a pharmaceutical acceptable excipient and/or carrier. Eye drops or an eye ointment or a formulation in form of eye drops or eye ointment can be prepared as known by a person skilled in the art. For example, the eye drops can be prepared using a tonisity agent as e.g. concentrated glycerin or sodium chloride, a buffer as e.g. sodium phosphate or sodium acetate, a surfactant as e.g. polyoxyethylene sorbitan monooleate or polyoxyl 40 stearate, a stabilizer as e.g. sodium citrate or sodium edetate and/or a preservative such as benzalkonium chloride or paraben. The pH of the eye drops is preferably within the range that is acceptable for ophthalmic preparation. For example, it may be in the range of from pH 4 to 8. The eye ointment can be prepared with a generally used base such as white soft paraffin or liquid paraffin.

In a preferred embodiment, the compound or a salt thereof or the pharmaceutical composition according to the present invention is formulated for intraocular administration, in particular in form of an implantable device, in particular a micro-drug-reservoir, for efficient and controlled ocular drug delivery, in particular intraocular drug delivery. When the reservoir is implanted e.g. into the eye, physicians can replenish the medication without removing the reservoir. Implantable devices can be prepared as known by a person skilled in the art. For example, a Port Delivery System may be implanted that is currently tested in a phase III clinical trial (ClinicalTrials.gov Identifier: NCT03677934).

Thus, the present invention refers also to an implantable device, in particular an implantable micro-drug delivery system, comprising a preferred compound as defined above or a salt thereof and a pharmaceutical acceptable excipient and/or carrier.

In a preferred embodiment, the compound or a salt thereof or the pharmaceutical composition according to the present invention is formulated for a topical application to the skin in the form of transdermal patches and similar drug-containing composite devices, in particular a microporous polymer film membrane or hydroalcoholic gel base, for efficient and controlled transdermal drug delivery. Transdermal patches can be prepared as known by a person skilled in the art. For example, in 1984, the first transdermal oestradiol system for female hormone replacement therapy (Alza Cooperation, Palo Alto, CA, USA) reached the US market (Good et al., 1985).

Thus, the present invention also refers to a skin ointment, skin gel or transdermal patch comprising a preferred compound as defined above or a salt thereof and a pharmaceutical acceptable excipient and/or carrier. A skin gel and a skin ointment can be prepared as known by a person skilled in the art. For example, metered-dose applicators can be used, exemplified by Elestrin^{®} (oestradiol 0.06% in a hydroalcoholic gel base; Meda Pharmaceuticals, Somerset, NJ, USA) packed as 100 doses each of 0.87 g gel and Divigel^{®} (Orion Corporation Pharm, Turku, Finland) packed as single use gel-filled sachets (0.25, 0.5 and 1.0 g gel-filled foil packets containing 0.25, 0.5 and 1 mg of oestradiol respectively).

In a preferred embodiment, the pharmaceutical pack, the eye drops, eye ointment, implantable device, the skin ointment, skin gel and/or transdermal patches as described above are for use according to the present invention as described herein.

The salt of the preferred compound as defined above of all embodiments of the present invention is preferably a pharmaceutical acceptable salt of the respective compound.

In a specific embodiment of the present invention, a preferred compound as defined above or a salt thereof and/or the pharmaceutical composition of the present invention is used as a medicament for the treatment of BEST1-related retinopathies, in particular the so-called bestrophinopathies which may be selected from the group consisting of autosomal dominant Best macular dystrophy, vitelliform macular dystrophy-2 (VMD2), autosomal dominant vitreoretinochoroidopathy (ADVIRC), autosomal recessive bestrophinopathy, or pattern dystrophy, more preferably from the group consisting of autosomal dominant Best macular dystrophy, vitelliform macular dystrophy-2 (VMD2), autosomal recessive bestrophinopathy, or pattern dystrophy. The treatment of autosomal dominant BVMD is especially preferred.

In another specific embodiment of the present invention the compound or a salt thereof and/or the pharmaceutical composition of the present invention is used in the manufacture of a medicament for the treatment of BEST1-related retinopathies, in particular the so-called bestrophinopathies which may be selected from the group consisting of autosomal dominant Best macular dystrophy, vitelliform macular dystrophy-2 (VMD2), autosomal dominant vitreoretinochoroidopathy (ADVIRC), autosomal recessive bestrophinopathy, or pattern dystrophy, more preferably from the group consisting of autosomal dominant Best macular dystrophy, vitelliform macular dystrophy-2 (VMD2), autosomal recessive bestrophinopathy, or pattern dystrophy. The treatment of autosomal dominant BVMD is especially preferred.

The following examples explain the present invention but are not considered to be limiting. It should be understood that the detailed description and specific examples disclosed herein, indicating particular embodiments of the invention, are given by way of illustration only.

### Example 1 - Generation of stable MDCKII cell lines

Transfection of MDCKII cells was performed with Lipofectamine 3000 transfection reagent following the manufacturer's (Thermofisher Scientific, Waltham, USA) instructions. MDCKII cells expressing wildtype and mutant BEST1 were cultured for 2 weeks in a selection medium containing 500 mg/ml G418 before single cell seeding in 96-well plates. 1-5 single clones of each cell line were selected.

### Example 2 - Generation of hiPSC-RPE cell lines from Best macular dystrophy patients

Skin biopsies were collected from Best macular dystrophy patients carrying disease associated mutations in the *BEST1* gene as well as from healthy control subjects carrying two normal *BEST1* gene copies. Adult human dermal fibroblasts were established and reprogrammed into human induced pluripotent stem cells (hiPSCs) via overexpression of transcription factors OCT3/4, Sox2, Klf4 and c-Myc (Takahashi et al., 2007). The cells were subsequently differentiated into hiPSC-derived RPE cells as described by Brandl et al. (2014).

### Example 3 - Immunofluorescence labeling

Cell monolayers were fixed in 4% paraformaldehyde (PFA)/PBS for 10 minutes and blocked by PBS containing 0.3% Triton X-100 and 10% goat serum for 25 min. Incubation with primary antibody against BEST1 and fluorescent-conjugated secondary antibody was performed at 4 °C ON. Immunolabelled hiPSC-RPE or MDCKII cells were imaged on a Zeiss confocal microscope LSM 510 (Zeiss, Göttingen, Germany).

### Example 4 - YFP-halide transport assay

Human hiPSC-RPE or MDCKII cells were transduced via lentivirus particles which were produced by co-transfecting HEK293T cells with the yellow fluorescence protein (YFPH148Q/I152L)-pLJM1 and helper plasmids pMD2.G and psPAX2 using the Ca²⁺ phospate transfection method. After six weeks of cultivation in 96-well black microtiter plates, cells were incubated with 40 mM Cl⁻ containing solution and basal YFP fluorescence was measured in a Tecan microplate reader. Subsequently Cl⁻ was replaced with equimolar I⁻ and decrease of YFP fluorescence intensity was monitored for another 2 min in 10 sec intervals. This assay can be performed in the reverse sequence where cells were pre-incubated with 40 mM I⁻ containing solution before replacing I⁻ by equimolar Cl⁻. Increases of YFP fluorescence intensity was monitored for another 7 min in 1 min intervals.

### Example 5 - Generation of MDCKII stable cell lines expressing normal and mutant BEST1

Initially, stable MDCKII cell lines constitutively expressing normal BEST1 and disease-associated BEST1 mutants (i.e. p.(T6P), p.(L21V), p.(W93C), p.(R218C), p.(L224M), p.(Y227N), p.(F305S)) were established. The mutants are located in the four mutational hotspots of BEST1 (White et al., 2000) (Fig. 1A). It was shown that polarized MDCKII cell lines are a well-suited model for studying protein trafficking and anion conductance in epithelial cells (Milenkovic et al., 2011; Milenkovic et al., 2018). Immunostaining for BEST1 with human polyclonal antibodies demonstrated protein localization to the plasma membrane for wildtype BEST1 and mutant BEST1-R218C, whereas the remaining 6 mutant proteins predominantly accumulated in the cytoplasm (Fig. 1B).

### Example 6 - Analysis of BEST1-mediated anion transport in cell lines

To analyze BEST1-mediated anion transport in the cell lines generated in the previous examples, a well-established halide transport assay (Galietta et al., 2001) was adapted. All cell lines were virally transduced with the yellow YFP-based halide sensor YFP(H148Q/I152L) and seeded on black 96-well plates revealing a bright and uniform cell fluorescence highly sensitive to iodide ions (I⁻) (Jayaraman et al., 1999) (Fig. 2A). Addition of I⁻ leads to specific YFP quenching over time and changes of fluorescence intensities can be monitored on a plate reader (Fig. 2B). MDCKII or hiPSC-RPE cells were incubated with 40 mM Cl⁻ containing solution and steady state YFP fluorescence signal intensities were measured after 6 minutes. Subsequently, Cl⁻ was replaced by equimolar I⁻ and anion permeability was monitored by decreasing YFP intensities as a result of BEST1-mediated I⁻ influx in a time course up to 2 minutes (Fig. 2C left graph). For several independent BEST1 wildtype cell lines a fast kinetic of decreasing fluorescence intensities to ~ 30 to 40% was observed while the untransfected control cell lines remained flat (Fig. 2C left graph). We also performed this assay in the reverse sequence where cells were pre-incubated with 40 mM I⁻ containing solution before replacing I⁻ by equimolar Cl⁻ (Fig. 2C right graph). To demonstrate the utility of this assay for characterizing channel function of BEST1, all Best macular dystrophy associated cell lines were analyzed under identical conditions. No significant differences in fluorescence intensities of mutant cell lines were found when compared to untransfected controls, demonstrating strongly reduced channel activity in BEST1 mutants, while cell line expressing wildtype BEST1 showed a fast kinetic of decreasing fluorescence signals as a result of BEST1-mediated ionic flux (Fig. 2D). Together, these data demonstrate that the assay is a suitable system for testing whether a compound is acting on BEST1 ion channels by either enhancing residual BEST1 channel function (potentiators) or activating other channels than BEST1 compensating its reduced anion transport activity.

### Example 7 - Evaluation of isoflavone derivatives effects on BEST1 transport function in MDCKII cells

MDCKII cells co-expressing YFP and BEST1 wildtype or mutant p.(R218C) were seeded on black 96-well plates and cultivated for 6 days to achieve polarity, a requirement for correct basolateral localization of BEST1 in epithelial cells. Compounds were tested separately at 10 µM concentration in a 96-well format. After compound addition for 24 h, cells were subjected to an outwardly directed I⁻-gradient to drive I⁻ efflux and produce increasing fluorescence signals. Each assay consisted of recording of base-line fluorescence for 10 seconds (= 100% value), followed by 7 min of recording of increasing fluorescence after addition of the Cl⁻-containing solution. Figure 3 shows a schematic of the screening approach. The effect of three isoflavone derivatives on the kinetics of Cl⁻ efflux in BEST1-R218C MDCKII cells is shown in Figure 5. Mutant BEST1-R218C showed no effect upon delivery of Biochanin A-7-isopropylate (Fig. 5A), while anion transport activity was greatly increased by the addition of Biochanin A diisopropylate (Fig. 5B). This activity could be further enhanced by Genistein triisopropylate (Fig. 5C).

### Example 8 - Evaluation of biological activity of several isoflavone derivatives in hiPSC-RPE cells generated from a patient with Best macular dystrophy

Next, it was tested whether the results from the fluorescence-based assay in MDCKII cell lines can be verified in a cell line-based disease-relevant model system. To this end, iPSC-RPEs from skin fibroblasts of a healthy donor (hiPSC-RPE +/+) and a Best macular dystrophy patient heterozygous for BEST1 mutation p.(R218C) (hiPSC-RPE-R218C/+) was generated. Subsequently, RPE cells from +/+ and R218C/+ were lentivirally transduced with YFP. After 4 weeks of cultivation on transwell filters, cells were seeded on black 96-well plates and cultivated for another 2 weeks to achieve polarity, a crucial requirement for correct basolateral localization of BEST1 in epithelial cells. Compounds were tested at 20 µM concentration in a 96-well format. After compound addition for 24 h, cells were subjected to an outwardly directed iodid (I⁻)-gradient to drive BEST1-mediated I⁻ efflux and produce increasing fluorescence signals. Each assay consisted of recording of YFP quenching after I⁻ pre-incubation for 6 min (= 100% value), followed by 7 min of recording of increasing fluorescence after addition of the chloride (Cl⁻) containing solution. Changes of fluorescence intensities were monitored on a plate reader. The effect on the kinetics of I⁻ efflux was tested for Genistein (Fig. 7A), Genistein triacetate (Fig. 7B), Genistein triisopropylate (Fig. 7C), Genistein triacrylate (Fig. 7D), Genistein triisopropyl carbonate (Fig. 7E). Genistein pentanoate (Fig. 7F), 3',4'-O-Dimethylorobol diacetate (Fig. 8A), 3',4'-O-Dimethylorobol diisopropylate (Fig. 8B), Pratensein triacetate (Fig. 8C). While Genistein **showed no effect** upon addition of respective compound delivery in comparison to untreated cells, anion transport activity was **strongly increased** upon addition of Genistein triacetate (Fig. 7B), Genistein triisopropylate (Fig. 7C), Genistein triacrylate (Fig. 7D), Genistein tri(isopropyl carbonate) (Fig. 7E), Genistein pentanoate (Fig. 7F), Dimethylorobol diacetate (Fig. 8A), 3',4'-O-Dimethylorobol diisopropylate (Fig. 8B) and Pratensein triacetate (Fig. 8C).

### References

Boon, C.J., Theelen, T., Hoefsloot, E.H. et al. (2009) Clinical and molecular genetic analysis of best vitelliform macular dystrophy. Retina 29: 835-847.
Brandl, C., Zimmermann, S. J., Milenkovic, V. M., Rosendahl, S. M., Grassmann, F., Milenkovic, A., Hehr, U., Federlin, M., Wetzel, C. H., Helbig, H. et al. (2014). In-Depth Characterisation of Retinal Pigment Epithelium (RPE) Cells Derived from Human Induced Pluripotent Stem Cells (hiPSC). Neuromolecular Med.
Burgess, R., Millar, I. D., Leroy, B. P., Urquhart, J. E., Fearon, I. M., De Baere, E., Brown, P. D., Robson, A. G., Wright, G. A., Kestelyn, P. et al. (2008). Biallelic mutation of BEST1 causes a distinct retinopathy in humans. Am J Hum Genet 82, 19-31.
Galietta, L. V., Jayaraman, S. and Verkman, A. S. (2001). Cell-based assay for high-throughput quantitative screening of CFTR chloride transport agonists. Am J Physiol Cell Physiol 281, C1734-42.
Good WR, Powers MS, Campbell P, Schenkel L. A new transdermal delivery system for estradiol. J Control Release. 1985;2:89-97.
Jayaraman, S., Teitler, L., Skalski, B. and Verkman, A. S. (1999). Long-wavelength iodide-sensitive fluorescent indicators for measurement of functional CFTR expression in cells. Am J Physiol 277, C1008-18.
Khan, K.N., Islam, F., Moore, A.T. et al. (2018) The fundus phenotype associated with the p.Ala243Val BEST1 mutation. Retina 38: 606-613.
Marquardt, A., Stohr, H., Passmore, L. A., Kramer, F., Rivera, A. and Weber, B. H. (1998). Mutations in a novel gene, VMD2, encoding a protein of unknown properties cause juvenile-onset vitelliform macular dystrophy (Best's disease). Hum Mol Genet 7, 1517-25.
Milenkovic, A., Milenkovic, V. M., Wetzel, C. H. and Weber, B. H. F. (2018). BEST1 protein stability and degradation pathways differ between autosomal dominant Best disease and autosomal recessive bestrophinopathy accounting for the distinct retinal phenotypes. Hum Mol Genet 27, 1630-1641.
Milenkovic, V. M., Rohrl, E., Weber, B. H. and Strauss, O. (2011). Disease-associated missense mutations in bestrophin-1 affect cellular trafficking and anion conductance. J Cell Sci 124, 2988-96.
Petrukhin, K., Koisti, M. J., Bakall, B., Li, W., Xie, G., Marknell, T., Sandgren, O., Forsman, K., Holmgren, G., Andreasson, S. et al. (1998). Identification of the gene responsible for Best macular dystrophy. Nat Genet 19, 241-7.
Takahashi, K., Tanabe, K., Ohnuki, M., Narita, M., Ichisaka, T., Tomoda, K. and Yamanaka, S. (2007). Induction of pluripotent stem cells from adult human fibroblasts by defined factors. Cell 131, 861-72.
White, K., Marquardt, A. and Weber, B. H. (2000). VMD2 mutations in vitelliform macular dystrophy (Best disease) and other maculopathies. Hum Mutat 15, 301-8.
Yardley, J., Leroy, B. P., Hart-Holden, N., Lafaut, B. A., Loeys, B., Messiaen, L. M., Perveen, R., Reddy, M. A., Bhattacharya, S. S., Traboulsi, E. et al. (2004). Mutations of VMD2 splicing regulators cause nanophthalmos and autosomal dominant vitreoretinochoroidopathy (ADVIRC). Invest Ophthalmol Vis Sci 45, 3683-9.

## Claims

1. A compound having the formula (I) or a salt thereof: 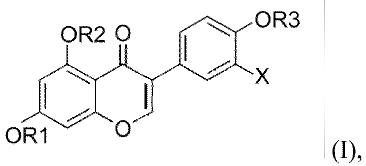 wherein
OR1 is an acetate, a pentanoate, an isopropylate, an acrylate, a methyl fumarate, a butyl carbamate, or an isopropyl carbonate,
OR2 is an acetate, a pentanoate, an isopropylate, an acrylate, a methyl fumarate, a butyl carbamate, or an isopropyl carbonate,
OR3 is an acetate, a pentanoate, an isopropylate, an acrylate, a methyl fumarate, a butyl carbamate, or an isopropyl carbonate, or R3 is a methyl,
X is a hydrogen or OR4, wherein OR4 is an acetate, a pentanoate, an isopropylate, an acrylate, a methyl fumarate, a butyl carbamate or an isopropyl carbonate, or R4 is a methyl,
under the provision that when X is a hydrogen, R3 is not a methyl,
for use in a method of treating or preventing BEST1-related retinopathies such as autosomal dominant Best vitelliform macular dystrophy.

2. The compound or a salt thereof for use according to claim 1, wherein R1 and R2 are the same.

3. The compound or a salt thereof for use according to claim 1 or 2, wherein
(i) X is a hydrogen and R1, R2 and R3 are preferably the same, or
(ii) X is O-R4 and R4 is a methyl and R1, R2 and R3 are preferably the same, or
(iii) R3 is a methyl, X is OR4 and R4 is a methyl, or
(iv) X is OR4 and R1, R2, R3 and R4 are preferably the same, or
(v) R3 is a methyl and X is OR4 and R1, R2 and R4 are preferably the same.

4. The compound or a salt thereof for use according to any one of the proceeding claims, wherein the compound is selected from the group consisting of: Genistein triacetate, Genistein tripentanoate, Genistein triisopropylate, Genistein triacrylate, Genistein tri(methyl fumarate), Genistein tri(butyl carbamate), Genistein tri(isopropyl carbonate), Pratensein triacetate, Pratensein tripentanoate, Pratensein triisopropylate, Pratensein triacrylate, Pratensein tri(methyl fumarate), Pratensein tri(butyl carbamate), Pratensein tri(isopropyl carbonate), 3'-O-Methylorobol triacetate, 3'-O-Methylorobol tripentanoate, 3'-O-Methylorobol triisopropylate, 3'-O-Methylorobol triacrylate, 3'-O-Methylorobol tri(methyl fumarate), 3'-O-Methylorobol tri(butyl carbamate), 3'-O-Methylorobol tri(isopropyl carbonate), 3',4'-O-Dimethylorobol diacetate, 3',4'-O-Dimethylorobol dipentanoate, 3',4'-O-Dimethylorobol diisopropylate, 3',4'-*O-*Dimethylorobol diacrylate, 3',4'-O-Dimethylorobol di(methyl fumarate), 3',4'-*O-*Dimethylorobol di(butyl carbamate), 3',4'-O-Dimethylorobol di(isopropyl carbonate), 5,7,3',4'-O-Orobol tetraacetate, 5,7,3',4'-O-Orobol tetrapentanoate, *5,7,3',4'-O-*Orobol tetraisopropylate, 5,7,3',4'-O-Orobol tetraacrylate, 5,7,3',4'-*O*-Orobol tetra(methyl fumarate), 5,7,3',4'-O-Orobol tetra(butyl carbamate), 5,7,3',4'-*O*-Orobol tetra(isopropyl carbonate).

5. The compound or a salt thereof for use according to any one of the proceeding claims, wherein preventing BEST1-related retinophathies such as autosomal dominant Best vitelliform macular dystrophy comprises delaying the onset or progression of BEST1-related retinopathies such as autosomal dominant Best vitelliform macular dystrophy.

6. The compound or a salt thereof for use according to any one of the proceeding claims, wherein the compound or a salt thereof is administered in an amount sufficient to (i) improve chloride conductance across the basolateral membrane of the retinal pigment epithelium, and/or (ii) restore BEST1 channel function.

7. The compound or a salt thereof for use according to any one of the proceeding claims, wherein the compound or a salt thereof is administered in an amount sufficient for activating other channels than BEST1 for compensating impaired anion transport activity of BEST1.

8. The compound or a salt thereof for use according to any one of the proceeding claims, wherein the method of treating or preventing comprises the steps of
(i) evaluating the subject's vision before treatment;
(ii) molecular genetic testing of the *BEST1* gene;
(iii) administering the compound according to formula (I) as defined in any one of the proceeding claims or a salt thereof to the subject; and
(iv) evaluating of subject's vision after step (iii).

9. The compound or a salt thereof for use according to any one of the proceeding claims, wherein the compound or the salt thereof is formulated for oral, ocular, intraocular, and/or topical administration, in particular for topical application to the eye or topical application to the skin.

10. The compound or a salt thereof for use according to any one of the proceeding claims, wherein the compound or a salt thereof is formulated in form of eye drops, an eye ointment, a transdermal patch, an ointment, a gel, a film, a tablet, a capsule, a dragee, a pill, an injectable solution or an implantable device.

11. A pharmaceutical composition comprising a compound according to formula (I) as defined in any one of the proceeding claims or salt thereof and a pharmaceutical acceptable excipient and/or carrier for use in a method of treating or preventing BESTl-related retinopathies such as autosomal dominant Best vitelliform macular dystrophy.

12. The compound or a salt thereof for use according to any one of claims 1 to 10 or the pharmaceutical composition for use according to claim 11, wherein the BESTl-related retinopathies are bestrophinopathies and/or wherein the BEST1-related retinopathies are **characterized by** an impaired and/or reduced anion transport activity of BEST1.

13. The compound or a salt thereof for use according to any one of claims 1 to 10 or 12 or the pharmaceutical composition for use according to claim 11 or 12, wherein the BEST1-related retinopathies are selected from the group consisting of autosomal dominant Best macular dystrophy, autosomal recessive bestrophinopathy, or pattern dystrophy, in particular pattern dystrophy due to BEST1 mutation p.(Ala243Val).

14. Eye drops, an eye ointment, a skin ointment, a skin gel, a transdermal patch or an implantable device, in particular a micro-drug delivery system, for use according to any of claims 1 to 13 comprising (i) a compound as defined in any one of claims 1 to 10 or 12 to 13 or (ii) a pharmaceutical composition as defined in any of claims 11 to 13.

15. A pharmaceutical pack for use according to any of claims 1 to 13 comprising one or more compartments, wherein at least one compartment comprises (i) a compound or salt thereof as defined in any one of claims 1 to 10 or 12 to 13 or (ii) a pharmaceutical composition as defined in any of claims 11 to 13.

## Patentansprüche

1. Eine Verbindung der Formel (I) oder ein Salz davon: 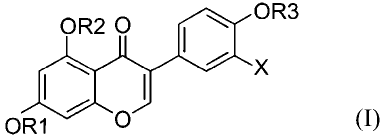 wobei
OR1 ein Acetat, ein Pentanoat, ein Isopropylat, ein Acrylat, ein Methylfumarat, ein Butylcarbamat oder ein Isopropylcarbonat ist,
OR2 ein Acetat, ein Pentanoat, ein Isopropylat, ein Acrylat, ein Methylfumarat, ein Butylcarbamat oder ein Isopropylcarbonat ist,
OR3 ein Acetat, ein Pentanoat, ein Isopropylat, ein Acrylat, ein Methylfumarat, ein Butylcarbamat oder ein Isopropylcarbonat ist oder R3 ein Methyl ist,
X ein Proton oder OR4 ist, wobei OR4 ein Acetat, ein Pentanoat, ein Isopropylat, ein Acrylat, ein Methylfumarat, ein Butylcarbamat oder ein Isopropylcarbonat ist oder R4 ein Methyl ist,
unter der Voraussetzung, dass wenn X ein Proton ist, R3 kein Methyl ist,
zur Verwendung in einem Verfahren zur Behandlung oder Vorbeugung von BEST1-assoziierten Retinopathien wie beispielsweise der autosomal-dominanten Best-Vitelliform-Makuladystrophie.

2. Die Verbindung oder ein Salz davon zur Verwendung nach Anspruch 1, wobei R1 und R2 gleich sind.

3. Die Verbindung oder ein Salz davon zur Verwendung nach Anspruch 1 oder 2, wobei
(i) X ein Proton ist und R1, R2 und R3 vorzugsweise gleich sind, oder
(ii) X ist O-R4 und R4 ist ein Methyl und R1, R2 und R3 sind vorzugsweise gleich, oder
(iii) R3 ein Methyl ist, X ist OR4 und R4 ist ein Methyl, oder
(iv) X ist OR4 und R1, R2, R3 und R4 sind vorzugsweise gleich, oder
(v) R3 ein Methyl ist und X ist OR4 und R1, R2 und R4 sind vorzugsweise gleich.

4. Die Verbindung oder ein Salz davon zur Verwendung nach einem der vorhergehenden Ansprüche, wobei die Verbindung ausgewählt ist aus der Gruppe bestehend aus: Genistein-triacetat, Genistein-tripentanoat, Genistein-triisopropylat, Genistein-triacrylat, Genistein-tri(methylfumarat), Genistein-tri(butylcarbamat), Genistein-tri(isopropylcarbonat), Pratensein-triacetat, Pratensein-tripentanoat, Pratensein-triisopropylat, Pratensein-triacrylat, Pratensein-tri(methylfumarat), Pratensein-tri(butylcarbamat), Pratensein-tri(isopropylcarbonat), 3'-O-Methylorobol-triacetat, 3'-O-Methylorobol-tripentanoat, 3'-O-Methylorobol-triisopropylat, 3'-O-Methylorobol-triacrylat, 3'-O-Methylorobol-tri(methylfumarat), 3'-O-Methylorobol-tri(butylcarbamat), 3'-O-Methylorobol-tri(isopropylcarbonat), 3',4'-O-Dimethylorobol-diacetat, 3',4'-O-Dimethylorobol-dipentanoat, 3',4'-O-Dimethylorobol-diisopropylat, 3',4'-O-Dimethylorobol-diacrylat, 3',4'-O-Dimethylorobol-di(methylfumarat), 3',4'-O-Dimethylorobol-di(butylcarbamat), 3',4'-O-Dimethylorobol-di(isopropylcarbonat), 5,7,3',4'-O-Orobol-tetraacetat, 5,7,3',4'-O-Orobol-tetrapentanoat, 5,7,3',4'-O-Orobol-tetraisopropylat, 5,7,3',4'-O-Orobol-tetraacrylat, 5,7,3',4'-O-Orobol-tetra(methylfumarat), 5,7,3',4'-O-Orobol-tetra(butylcarbamat), 5,7,3',4'-O-Orobol-tetra(isopropylcarbonat).

5. Die Verbindung oder ein Salz davon zur Verwendung nach einem der vorhergehenden Ansprüche, wobei die Vorbeugung von BEST1-assoziierten Retinopathien wie der autosomal-dominanten Best-Vitelliform-Makuladystrophie das Verzögern des Ausbruchs oder des Fortschreitens von BEST1-assoziierten Retinopathien wie der autosomal-dominanten Best-Vitelliform-Makuladystrophie umfasst.

6. Die Verbindung oder ein Salz davon zur Verwendung nach einem der vorhergehenden Ansprüche, wobei die Verbindung oder ein Salz davon in einer Menge verabreicht wird, die ausreichend ist, um (i) die Chloridleitfähigkeit über die basolaterale Membran des retinalen Pigmentepithels zu verbessern und/oder (ii) die BEST1-Kanalfunktion wiederherzustellen.

7. Die Verbindung oder ein Salz davon zur Verwendung nach einem der vorhergehenden Ansprüche, wobei die Verbindung oder ein Salz davon in einer Menge verabreicht wird, die ausreichend ist, um andere Kanäle als BEST1 zu aktivieren, um die beeinträchtigte Anionentransportaktivität von BEST1 zu kompensieren.

8. Die Verbindung oder ein Salz davon zur Verwendung nach einem der vorhergehenden Ansprüche, wobei das Verfahren zur Behandlung oder Vorbeugung die folgenden Schritte umfasst:
(i) Evaluieren des Sehvermögens des Patienten vor der Behandlung;
(ii) molekulargenetische Untersuchung des BEST1-Gens;
(iii) Verabreichung der Verbindung nach Formel (I), wie in einem der vorhergehenden Ansprüche definiert, oder eines Salzes davon an den Patienten; und
(iv) Evaluieren des Sehvermögens des Patienten nach Schritt (iii).

9. Die Verbindung oder ein Salz davon zur Verwendung nach einem der vorhergehenden Ansprüche, wobei die Verbindung oder das Salz davon zur oralen, okulären, intraokulären und/oder topischen Anwendung formuliert ist, insbesondere zur topischen Anwendung am Auge oder zur topischen Anwendung auf der Haut.

10. Die Verbindung oder ein Salz davon zur Verwendung nach einem der vorhergehenden Ansprüche, wobei die Verbindung oder ein Salz davon in Form von Augentropfen, einer Augensalbe, eines transdermalen Pflasters, einer Salbe, eines Gels, eines Films, einer Tablette, einer Kapsel, einer Dragee, einer Pille, einer injizierbaren Lösung oder einer implantierbaren Vorrichtung formuliert ist.

11. Eine pharmazeutische Zusammensetzung umfassend eine Verbindung nach Formel (I), wie in einem der vorhergehenden Ansprüchen definiert, oder ein Salz davon und einen pharmazeutisch geeigneten Hilfsstoff und/oder Träger zur Verwendung in einem Verfahren zur Behandlung oder Vorbeugung von BEST1-assoziierten Retinopathien, wie beispielsweise der autosomal-dominanten Best-Vitelliform-Makuladystrophie.

12. Die Verbindung oder ein Salz davon zur Verwendung nach einem der Ansprüche 1 bis 10 oder die pharmazeutische Zusammensetzung zur Verwendung nach Anspruch 11, wobei die BEST1-assoziierten Retinopathien Bestrophinopathien sind und/oder wobei die BEST1-assoziierten Retinopathien durch eine beeinträchtigte und/oder reduzierte Anionentransportaktivität von BEST1 gekennzeichnet sind.

13. Die Verbindung oder ein Salz davon zur Verwendung nach einem der Ansprüche 1 bis 10 oder 12 oder die pharmazeutische Zusammensetzung zur Verwendung nach den Ansprüchen 11 oder 12, wobei die BEST1- assoziierten Retinopathien ausgewählt sind aus der Gruppe bestehend aus autosomal-dominanter Best-Makuladystrophie, autosomal-rezessiver Bestrophinopathie oder Musterdystrophie, insbesondere Musterdystrophie aufgrund einer BEST1-Mutation p.(Ala243Val).

14. Augentropfen, eine Augensalbe, eine Hautsalbe, ein Hautgel, ein transdermales Pflaster oder eine implantierbare Vorrichtung, insbesondere ein Mikro-Arzneimittelabgabesystem, zur Verwendung nach einem der Ansprüche 1 bis 13, umfassend (i) eine Verbindung nach einem der Ansprüche 1 bis 10 oder 12 bis 13 oder (ii) eine pharmazeutische Zusammensetzung nach einem der Ansprüche 11 bis 13 .

15. Eine pharmazeutische Verpackung zur Verwendung nach einem der Ansprüche 1 bis 13, umfassend ein oder mehrere Abschnitte, wobei mindestens ein Abschnitt (i) eine Verbindung oder ein Salz davon, wie in einem der Ansprüche 1 bis 10 oder 12 bis 13 definiert, oder (ii) eine pharmazeutische Zusammensetzung, wie in einem der Ansprüche 11 bis 13 definiert, umfasst.

## Revendications

1. Composé de formule (I) ou un sel de celui-ci : 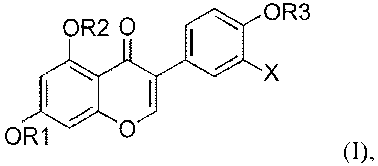 dans lequel
OR1 est un acétate, un pentanoate, un isopropylate, un acrylate, un méthylfumarate, un butylcarbamate, ou un isopropylcarbonate,
OR2 est un acétate, un pentanoate, un isopropylate, un acrylate, un méthylfumarate, un butylcarbamate, ou un isopropylcarbonate,
OR3 est un acétate, un pentanoate, un isopropylate, un acrylate, un méthylfumarate, un butylcarbamate, ou un isopropylcarbonate, ou bien R3 est un méthyle,
X est un hydrogène ou OR4, dans lequel OR4 est un acétate, un pentanoate, un isopropylate, un acrylate, un méthylfumarate, un butylcarbamate, ou un isopropylcarbonate, ou bien R4 est un méthyle,
sous réserve que, lorsque X est un hydrogène, alors R3 ne soit pas un méthyle,
pour une utilisation dans une méthode de traitement ou de prévention de rétinopathies associées à BEST1, telles que la dystrophie maculaire vitelliforme de Best autosomique dominante.

2. Composé ou un sel de celui-ci pour une utilisation selon la revendication 1, dans lequel R1 et R2 sont identiques.

3. Composé ou un sel de celui-ci pour une utilisation selon la revendication 1 ou 2, dans lequel
(i) X est un hydrogène et R1, R2 et R3 sont de préférence identiques, ou
(ii) X est O-R4 et R4 est un méthyle et R1, R2 et R3 sont de préférence identiques, ou
(iii) R3 est un méthyle, X est OR4 et R4 est un méthyle, ou
(iv) X est OR4 et R1, R2, R3 et R4 sont de préférence identiques, ou
(v) R3 est un méthyle et X est OR4 et R1, R2 et R4 sont de préférence identiques.

4. Composé ou un sel de celui-ci pour une utilisation selon l'une quelconque des revendications précédentes, dans lequel le composé est choisi dans le groupe constitué par : le triacétate de génistéine, le tripentanoate de génistéine, le triisopropylate de génistéine, le triacrylate de génistéine, le tri(méthylfumarate) de génistéine, le tri(butylcarbamate) de génistéine, le tri(isopropylcarbonate) de génistéine, le triacétate de pratenséine, le tripentanoate de pratenséine, le triisopropylate de pratenséine, le triacrylate de pratenséine, le tri(méthylfumarate) de pratenséine, le tri(butylcarbamate) de pratenséine, le tri(isopropylcarbonate) de pratenséine, le triacétate de 3'-*O*-méthylorobol, le tripentanoate de 3'-*O*-méthylorobol, le triisopropylate de 3'-*O*-méthylorobol, le triacrylate de 3'-*O*-méthylorobol, le tri(méthylfumarate) de 3'-*O*-méthylorobol, le tri(butylcarbamate) de 3'-*O*-méthylorobol, le tri(isopropylcarbonate) de 3'-*O-*méthylorobol, le diacétate de 3',4'-*O*-diméthylorobol, le dipentanoate de 3',4'-*O-*diméthylorobol, le diisopropylate de 3',4'-*O*-diméthylorobol, le diacrylate de 3',4'-*O-*diméthylorobol, le di(méthylfumarate) de 3',4'-*O*-diméthylorobol, le di(butylcarbamate) de 3',4'-*O*-diméthylorobol, le di(isopropylcarbonate) de 3',4'-*O*-diméthylorobol, le tétraacétate de 5,7,3',4'-*O*-orobol, le tétrapentanoate de 5,7,3',4'-*O*-orobol, le tétraisopropylate de 5,7,3',4'-*O*-orobol, le tétraacrylate de 5,7,3',4'-*O*-orobol, le tétra(méthylfumarate) de 5,7,3',4'-*O*-orobol, le tétra(butylcarbamate) de 5,7,3',4'-*O-*orobol, le tétra(isopropylcarbonate) de 5,7,3',4'-*O*-orobol.

5. Composé ou un sel de celui-ci pour une utilisation selon l'une quelconque des revendications précédentes, dans lequel la prévention de rétinopathies associées à BEST1 telles que la dystrophie maculaire vitelliforme de Best autosomique dominante comprend le retardement de l'apparition ou de la progression de rétinopathies associées à BEST1 telles que la dystrophie maculaire vitelliforme de Best autosomique dominante.

6. Composé ou un sel de celui-ci pour une utilisation selon l'une quelconque des revendications précédentes, dans lequel le composé ou sel de celui-ci est administré en une quantité suffisante pour (i) améliorer la conductance du chlorure à travers la membrane basolatérale de l'épithélium pigmentaire rétinien, et/ou (ii) restaurer la fonction de canal de BEST1.

7. Composé ou un sel de celui-ci pour une utilisation selon l'une quelconque des revendications précédentes, dans lequel le composé ou sel de celui-ci est administré en une quantité suffisante pour activer d'autres canaux que BEST1 pour compenser l'activité de transport d'anions dégradée de BEST1.

8. Composé ou un sel de celui-ci pour une utilisation selon l'une quelconque des revendications précédentes, dans lequel la méthode de traitement ou de prévention comprend les étapes de
(i) évaluation de la vision du sujet avant le traitement ;
(ii) test génétique moléculaire du gène *BEST1* ;
(iii) administration du composé de formule (I) tel que défini dans l'une quelconque des revendications précédentes ou d'un sel de celui-ci au sujet ; et
(iv) évaluation de la vision du sujet après l'étape (iii).

9. Composé ou un sel de celui-ci pour une utilisation selon l'une quelconque des revendications précédentes, dans lequel le composé ou le sel de celui-ci est formulé pour une administration par voie orale, oculaire, intraoculaire et/ou topique, en particulier pour une application topique à l'œil ou une application topique à la peau.

10. Composé ou un sel de celui-ci pour une utilisation selon l'une quelconque des revendications précédentes, dans lequel le composé ou sel de celui-ci est formulé sous la forme de gouttes oculaires, de pommade oculaire, de timbre transdermique, de pommade, de gel, de film, de comprimé, de capsule, de dragée, de pilule, de solution injectable ou de dispositif implantable.

11. Composition pharmaceutique comprenant un composé de formule (I) tel que défini dans l'une quelconque des revendications précédentes ou un sel de celui-ci et un excipient et/ou véhicule pharmaceutiquement acceptable, pour une utilisation dans une méthode de traitement ou de prévention de rétinopathies associées à BEST1 telles que la dystrophie maculaire vitelliforme de Best autosomique dominante.

12. Composé ou un sel de celui-ci pour une utilisation selon l'une quelconque des revendications 1 à 10 ou composition pharmaceutique pour une utilisation selon la revendication 11, dans lequel les rétinopathies associées à BEST1 sont des bestrophinopathies et/ou dans lequel les rétinopathies associées à BEST1 sont **caractérisées par** une activité de transport d'anions dégradée et/ou réduite de BEST1.

13. Composé ou un sel de celui-ci pour une utilisation selon l'une quelconque des revendications 1 à 10 ou 12 ou composition pharmaceutique pour une utilisation selon la revendication 11 ou 12, dans lequel les rétinopathies associées à BEST1 sont choisies dans le groupe constitué par la dystrophie maculaire vitelliforme de Best autosomique dominante, la bestrophinopathie autosomique récessive, ou la dystrophie à motifs, en particulier la dystrophie à motifs due à la mutation p.(Ala243Val) de BEST1.

14. Gouttes oculaires, pommade oculaire, pommade cutanée, gel cutané, timbre transdermique ou dispositif implantable, en particulier système de micro-administration de médicament, pour une utilisation selon l'une quelconque des revendications 1 à 13, comprenant (i) un composé tel que défini dans l'une quelconque des revendications 1 à 10 ou 12 à 13, ou (ii) une composition pharmaceutique telle que définie dans l'une quelconque des revendications 11 à 13.

15. Conditionnement pharmaceutique pour une utilisation selon l'une quelconque des revendications 1 à 13, comprenant un ou plusieurs compartiments, dans lequel au moins un compartiment comprend (i) un composé ou un sel de celui-ci tel que défini dans l'une quelconque des revendications 1 à 10 ou 12 à 13, ou (ii) une composition pharmaceutique telle que définie dans l'une quelconque des revendications 11 à 13.
